# EUROPEAN PATENT SPECIFICATION

(11) **EP 1 553 100 B1**
(45) Date of publication and mention of the grant of the patent: **21.07.2010**
(21) Application number: 03741117.0
(22) Date of filing: 16.06.2003
(51) Int. Cl.: C07H 15/04, C07H 1/00, A23L 1/00, A23L 1/09, A23L 1/236, A23P 1/12, C13F 1/02

(54) **PROCESS FOR PRODUCING CRYSTALLINE MALTITOL**
VERFAHREN ZUR HERSTELLUNG VON KRISTALLINEM MALTIT
PROCEDE DE PRODUCTION DE MALTITOL CRISTALLIN

(30) Priority: 19.06.2002 JP 2002178319
(43) Date of publication of application: 13.07.2005
(73) Proprietor: Ueno Fine Chemicals Industry, Ltd., Chuo-ku Osaka-shi Osaka 541-8543 (JP)
(72) Inventor: UENO, Ryuzo, Nishinomiya-shi, Hyogo 662-0038 (JP); HONDA, Junya 502, Nishinomiyanajio-, Nishinomiya-shi, Hyogo 669-1133 (JP); FURUKAWA, Yojiro, Hyogo 664-0847 (JP)
(74) Representative: Benson, John Everett
(86) International application number: PCT/JP2003/007613
(87) International publication number: WO 2004/000861

(56) References cited:
- EP-A1- 0 202 165
- EP-A2- 0 937 733
- JP-A- 6 234 786
- JP-A- 9 132 587
- US-A- 5 583 215

## Description

### Technical Field

The present invention relates to a production method of crystalline maltitol.

### Background Art

Maltitol is hardly digested and absorbed in digestive tracts and is hardly fermented by intraoral germs. Hence, it is used as a sweetener for low calorie foods, diet foods, low cariosity foods, diabetics and other applications. However, maltitol has a problem that since dried maltitol is significantly liable to absorb moisture and undergo deliquescence and therefore does not become powdery easily, it is inconvenient to handle.

To solve this problem, a number of techniques have been proposed for crystallization or pulverization of maltitol. Japanese Patent Publication No. 7-14953 proposes a method of producing a maltitol crystalline mixture solid by feeding an aqueous solution of maltitol continuously to an extruder having a long, narrow cooling and kneading zone, cooling and kneading the solution in the presence of seed crystals so as to produce maltitol magma, and extruding the maltitol magma continuously from an extrusion nozzle.

However, the production method using seed crystals has a problem that to improve a production rate, the amount of the seed crystals must be increased, and therefore a large amount of additional seed crystals must be prepared, thereby making a production process complicated.

### Disclosure of the Invention

An object of the present invention is to provide a novel production method of crystalline maltitol with significantly improved production efficiency as compared with that of the conventional production method of a maltitol crystalline mixture solid.

Another object of the present invention is to provide novel methods for producing crystalline maltitol without using seed crystals, with good workability, at low costs, and with efficiency in a short time.

Other objects and advantages of the present invention will be apparent from the following description.

According to the present invention, firstly, the above objects and advantages of the present invention are achieved by a method of producing (hereinafter occasionally referred to as "first invention") crystalline maltitol, comprising the steps of feeding a first maltitol aqueous solution to a kneader so as to knead and cool it, then feeding a second maltitol aqueous solution having a higher solid content than that of the first maltitol aqueous solution to the kneader and further kneading and cooling the resulting mixture so as to produce a plastic mass, cooling the produced plastic mass so as to solidify it, and pulverizing the solidified mass.

Further, according to the present invention, secondly, the above objects and advantages of the present invention are achieved by a method of producing (hereinafter occasionally referred to as "second invention") crystalline maltitol, comprising the steps of feeding a maltitol aqueous solution to a kneader so as to knead and cool it, then adding water to the kneader and further kneading and cooling the mixture so as to produce a plastic mass, cooling the produced plastic mass so as to solidify it, and pulverizing the solidified mass.

### Preferred Embodiment of the Invention

Hereinafter, the methods of the present invention will be described in detail.

In the first invention, to produce crystalline maltitol, at least two types of raw materials having different solid contents are used. More specifically, as the first maltitol aqueous solution (hereinafter referred to as "first raw material") which is fed to a kneader first, a raw material having a relatively low solid content is used, and as the second maltitol aqueous solution (hereinafter referred to as "second raw material") which is fed to the kneader subsequently to the first raw material, a raw material having a higher solid content than that of the first raw material is used. Thereby, it becomes possible to omit a conventionally commonly practiced step of preparing additional seed crystals and feeding the seed crystals continuously to the kneader. Although the solid contents of the first and second raw materials are not limited to particular solid contents, it is important that the first raw material has a lower solid content than the second raw material. For example, as the first raw material, a maltitol aqueous solution with a solid content of 70 to 97 wt% and a purity of not lower than 88 wt% is preferred, and a maltitol aqueous solution with a solid content of 93 to 96 wt% and a purity of not lower than 90 wt% is more preferred. Meanwhile, as the second raw material, a maltitol aqueous solution with a solid content of 97.5 to 99.5 wt% and a purity of not lower than 88 wt% is preferred, and a maltitol aqueous solution with a solid content of 98 to 99 wt% and a purity of not lower than 90 wt% is more preferred.

The feed rates of the first and second raw materials vary according to the type and capacity of a kneader used and must therefore be set as appropriate. The feed rates of the first and second raw materials should be set such that optimum production efficiency is achieved in each kneader. Further, the feed rates of the first and second raw materials do not have to be the same, and they may be fed as different rates. For example, when a KRC kneader S2 type (product of KURIMOTO LTD.) is used as a kneader, the feed rate of the first raw material is preferably about 0.1 to 6.0 kg/hr, and the feed rate of the second raw material is preferably about 0.1 to 15 kg/hr.

Further, the jacket temperature of the kneader at the time of feeding of the raw materials should be set at a temperature at which generated heat of crystallization can be removed. When a KRC kneader S2 type (product of KURIMOTO LTD.) is used, it is preferably not higher than 110°C, more preferably not higher than 95° C. This jacket temperature may be set at the time of feedings of the first and second raw materials according to the degree of generation of heat of crystallization.

The temperatures at which the raw materials are fed to the kneader are preferably temperatures at which maltitol crystals are not deposited. In consideration of the fact that higher flowability makes handling easier and ease of adjustment in forming a plastic mass, the temperature for the first raw material is preferably about 90 to 120°C, and the temperature for the second raw material is preferably 120 to 140°C.

In the first invention, switching from the first raw material to the second raw material is preferably performed after production of a white, opaque plastic mass is observed inside the kneader or at the outlet of the kneader after the first raw material is put in the kneader. This switching causes an improvement in the production efficiency of crystalline maltitol.

In the second invention, there is no need to use two types of raw materials as in the first invention, and crystalline maltitol can be produced with only one type of raw material. In that case, as the raw material, a maltitol aqueous solution with a solid content of 97.5 to 99.5 wt% and a purity of not lower than 88 wt% is preferred, and a maltitol aqueous solution with a solid content of 98 to 99 wt% and a purity of not lower than 90 wt% is more preferred.

The feed rate of the maltitol aqueous solution used as the raw material is the same as that of the first raw material in the first invention. To further improve the production efficiency of crystalline maltitol, it is possible to change the feed rate of the raw material to the same rate as that of the second raw material in the first invention after a step of adding water which will be described later. Switching between the feed rates of the raw material is preferably performed after production of a white, opaque plastic mass is observed inside the kneader or at the outlet of the kneader.

In the second invention, as described above, water is added after the raw material is fed. The water may be service water, for example. However, to produce pure, clean crystal maltitol, ion exchanged water is preferably used.

Water is preferably added in an amount of about 1.0 to 7.0 parts by weight based on 100 parts by weight of the maltitol aqueous solution which is a raw material. Water can be added several times to 10 times plus a few more times according to the extent of production of crystalline maltitol. Further, water can be charged into the kneader continuously or intermittently by use of a pump or the like. Timing of addition of water differs depending on a kneader used. Water should be added after discharge of the raw material solution from the outlet is observed after the raw material is fully dispersed in the kneader.

Further, the jacket temperature of the kneader is the same as that in the first invention. However, it may be changed before or after the step of adding water.

In the present invention, the raw material is prepared by concentrating a maltitol aqueous solution having a solid content of about 70 wt%. In this case, the maltitol aqueous solution is preferably concentrated under a reduced pressure because the solution may be colored when it is heated under normal pressure so as to remove water. Since use of a colored raw material may interfere with production of target crystalline maltitol, it is important to prepare as colorless and transparent a raw material as possible.

A kneader usable in the present invention is not particularly limited and may be any of open, closed, batch and continuous kneaders as long as it is capable of performing kneading and cooling simultaneously. The kneader is preferably one capable of discharging a product continuously from the outlet after completion of kneading and cooling. Illustrative examples of such a kneader include an extruder, a continuous kneader, a mixtron and a kneadex. Of these, the extruder is more preferably used. Specific examples of the extruder include extruders such as a KRC kneader (product of KURIMOTO Ltd.), a twin screw extruder for food (product of JSW), and a twin screw cooking extruder (product of W & P Co., Ltd., Germany).

When a plastic mass is discharged from a continuous kneader, it can be in any form such as a noodle, a ribbon, a stick or a plate. In consideration of subsequent steps such as cooling and pulverization, the plastic mass is preferably discharged in the form of a noodle or ribbon. In that case, as a punching plate disposed at the outlet, one having a pore diameter of about 2 to 5 mm and a porosity of about 10 to 40% is preferably used.

A cooling method is not particularly limited. For example, a method comprising blowing cool air directly against a plastic mass discharged from a kneader, a method comprising leaving the mass to stand at room temperature or a method comprising cooling the mass to about room temperature on a metal mesh belt by cool air can be employed.

The obtained crystalline maltitol can be formed into powder or granules by pulverizing it without a drying step. A method of pulverization or granulation is not particularly limited. A commonly used pulverizer or granulator can be used. Further, if necessary, the obtained powder or granules may be dried by a commonly practiced drying method.

Hereinafter, the present invention will be described in more detail with reference to Examples and Comparative Examples.

### Examples

### Example 1

When a maltitol aqueous solution (purity = 90 wt%, solid content = about 95 wt%, 110°C, colorless and transparent) as a first raw material was continuously fed to a continuous kneader having a long, narrow kneading and cooling zone (KRC KNEADER S2 TYPE, product of KURIMOTO LTD., 60 rpm, jacket temperature = 80°C) at a rate of 4 kg/h and continuously kneaded and cooled, discharge of a white, opaque plastic mass from a punching plate at the outlet was observed after a while. Then, when another maltitol aqueous solution (purity = 90 wt%, solid content = about 98 wt%, 125°C, colorless and transparent) as a second raw material was continuously fed at a rate of 4 kg/h, a plastic mass in the form of a noodle was continuously discharged from the punching plate at the outlet. Then, although the feed rate of the second raw material was adjust at 8 kg/hr and the jacket temperature was adjust at 55°C, the plastic mass in the form of a noodle was discharged stably. This plastic mass was then cooled to 30°C or lower under blowing air so as to be solidified and then fed to POWERMILL P-3 TYPE (product of DALTON CO., LTD.) at 200 kg/hr so as to be pulverized. Thereby, crystalline maltitol of good quality was obtained.

### Example 2

A maltitol aqueous solution (purity = 92 wt%, solid content = about 97.6 wt%, 130°C, colorless and transparent) as a raw material was continuously fed to a continuous kneader having a long, narrow kneading and cooling zone (KRC KNEADER S2 TYPE, product of KURIMOTO LTD. , 60 rpm, jacket temperature = 80°C) at a rate of 4 kg/h. After 5 minutes from the start of feeding of the raw material, ion exchanged water was added at a rate of 0.12 kg/hr, and the mixture was continuously kneaded and cooled. As a result of stopping addition of water when discharge of a white, opaque plastic mass from a punching plate at the outlet was observed, a plastic mass in the form of a noodle was continuously discharged from the punching plate at the outlet. Then, although the feed rate of the raw material was adjust at 8 kg/hr and the jacket temperature was adjust at 55°C, the plastic mass in the form of a noodle was discharged stably. This plastic mass was then cooled to 30°C or lower under blowing air so as to be solidified and then fed to POWERMILL P-3 TYPE (product of DALTON CO. , LTD.) at 200 kg/hr so as to be pulverized. Thereby, crystalline maltitol of good quality was obtained.

As described above, according to the methods of the present invention, no seed crystals are used, and therefore crystalline maltitol can be produced with good workability, at low costs and with efficiency in a short time.

## Claims

1. A method of producing crystalline maltitol, comprising the steps of feeding a first maltitol aqueous solution to a kneader so as to knead and cool it, then feeding a second maltitol aqueous solution having a higher solid content than that of the first maltitol aqueous solution to the kneader and further kneading and cooling the resulting mixture so as to produce a plastic mass, cooling the produced plastic mass so as to solidify it, and pulverizing the solidified mass.

2. Method according to claim 1, wherein the first maltitol aqueous solution has a solid content of 70 to 97 wt%

3. Method according to claim 1 or 2, wherein the second maltitol aqueous solution has a solid content of 97.5 to 99.5 wt%.

4. A method of producing crystalline maltitol, comprising the steps of feeding a maltitol aqueous solution to a kneader so as to knead and cool it, then adding water to the kneader and further kneading and cooling the mixture so as to produce a plastic mass, cooling the produced plastic mass so as to solidify it, and pulverizing the solidified mass.

5. Method according to claim 4, wherein the maltitol aqueous solution has a solid content of 97.5 to 99.5 wt%.

6. Method according to claim 4 or claim 5, wherein water is added several times.

7. Method according to anyone of claims 1 to 6, wherein the kneader is a continuous kneading-extruder machine having a cooler.

## Patentansprüche

1. Verfahren zur Herstellung von kristallinem Maltit, umfassend die folgenden Stufen: Einspeisen einer ersten wässrigen Maltitlösung in einen Kneter, um es zu kneten und zu kühlen, anschließend Einspeisen einer zweiten wässrigen Maltitlösung, die einen höheren Feststoffgehalt als die erste wässrige Maltitlösung aufweist, in den Kneter und weiteres Kneten und Abkühlen des erhaltenen Gemisches, um eine plastische Masse zu erzeugen, Abkühlen der erzeugten plastischen Masse, um sie zu verfestigen, und Pulverisieren der verfestigten Masse.

2. Verfahren nach Anspruch 1, wobei die erste wässrige Maltitlösung einen Feststoffgehalt von 70 bis 97 Gew.-% aufweist.

3. Verfahren nach Anspruch 1 oder 2, wobei die zweite wässrige Maltitlösung einen Feststoffgehalt von 97,5 bis 99,5 Gew.-% aufweist.

4. Verfahren zur Herstellung von kristallinem Maltit, umfassend die folgenden Stufen: Einspeisen einer wässrigen Maltitlösung in einen Kneter, um es zu kneten und zu kühlen, anschließend Hinzugeben von Wasser zum Kneter und weiteres Kneten und Abkühlen des Gemisches, um eine plastische Masse zu erzeugen, Abkühlen der erzeugten plastischen Masse, um sie zu verfestigen, und Pulverisieren der verfestigten Masse.

5. Verfahren nach Anspruch 4, wobei die wässrige Maltitlösung einen Feststoffgehalt von 97,5 bis 99,5 Gew.-% aufweist.

6. Verfahren nach Anspruch 4 oder 5, wobei Wasser mehrmals zugegeben wird.

7. Verfahren nach einem der Ansprüche 1 bis 6, wobei es sich beim Kneter um eine kontinuierliche Kneter-Extruder-Maschine mit einem Kühler handelt.

## Revendications

1. Procédé de production de maltitol cristallin, comprenant les étapes de transférer une première solution aqueuse de maltitol vers un malaxeur de manière à la malaxer et la refroidir, puis transférer une seconde solution aqueuse de maltitol ayant une teneur en matières solides plus élevée que celle de la première solution aqueuse de maltitol vers le malaxeur et malaxer et refroidir de nouveau le mélange résultant de manière à produire une masse plastique, refroidir la masse plastique produite de manière à la solidifier, et pulvériser la masse solidifiée.

2. Procédé selon la revendication 1, dans lequel la première solution aqueuse de maltitol a une teneur en matières solides de 70 à 97 % en poids.

3. Procédé selon la revendication 1 ou 2, dans lequel la seconde solution aqueuse de maltitol a une teneur en matières solides de 97,5 à 99,5 % en poids.

4. Procédé de production de maltitol cristallin, comprenant les étapes de transférer une solution aqueuse de maltitol vers un malaxeur de manière à la malaxer et la refroidir, puis ajouter de l'eau au malaxeur et malaxer et refroidir de nouveau le mélange de manière à produire une masse plastique, refroidir la masse plastique produite de manière à la solidifier, et pulvériser la masse solidifiée.

5. Procédé selon la revendication 4, dans lequel la solution aqueuse de maltitol a une teneur en matières solides de 97,5 à 99,5 % en poids.

6. Procédé selon la revendication 4 ou la revendication 5, dans lequel de l'eau est ajoutée plusieurs fois.

7. Procédé selon l'une quelconque des revendications 1 à 6, dans lequel le malaxeur est une machine à malaxer-extruder continue ayant un refroidisseur.
